Europäisches Patentamt

(19) European Patent Office    (11) Publication number: **0 000 738**
Office européen des brevets    **B1**

(12)    # EUROPEAN PATENT SPECIFICATION

(45) Date of publication of patent specification:    (51) Int. Cl.³: **A 61 L  9/20**
08.04.81

(21) Application number: **78100517.8**

(22) Date of filing: **27.07.78**

---

(54) **An air purifier of the regenerating type.**

---

(30) Priority: **04.08.77  DK 3499/77**

(43) Date of publication of application:
**21.02.79 Bulletin 79/4**

(45) Publication of the grant of the patent:
**08.04.81 Bulletin 81/14**

(84) Designated Contracting States:
**BE CH DE FR GB LU NL SE**

(56) References cited:
**FR-A-2 227 017**
**FR-A-2 240 021**

(73) Proprietor: **Brundbjerg, Niels, Resedavej 12,
DK-2820 Gentofte (DK)**

(72) Inventor: **Brundbjerg, Niels, Resedavej 12,
DK-2820 Gentofte (DK)**

(74) Representative: **Goddar, Heinz, Dr. et al, FORRESTER &
BOEHMERT Widenmayerstrasse 5/IV,
D-8000 München 22 (DE)**

BUNDESDRUCKEREI BERLIN

## An air purifier of the regenerating type

The invention relates to an air purifier of the regenerating type, which comprises a radiating means such as a mercury lamp, and in which the air flows through an electric field in an electron-cloud forming member.

An air purifier comprising a mercury vapour lamp is known from, for example, British patent specification No. 1,400,519. The irradiation with ultraviolet rays changes the nature of the passing pollutant.

It is also known to treat and purify air by passing the air through an electrostatic field. As a result ozone is, however, produced, cf. e.g. the German Offenlegungsschrift Nos. 2,452,824 and 2,205,885. $O_3$ can be reduced to $O_2$ oxidizing a metallic material, cf. the above-mentioned British patent specification. This filter method is, however, not particularly efficient. Furthermore, the filter material must be replaced from time to time.

The invention as claimed is intended to provide an air purifier which is able to sterilize air and neutralize the ozone molecules in a more efficient manner.

The air purifier according to the invention is characterised by the radiating means being surrounded by a negative charge absorbing, non-conductive casing.

As a result the ozone molecules become positively charged, whereby a reaction with negatively charged ozone molecules and consequently a neutralisation is facilitated.

According to a preferred embodiment of the invention the non-conductive casing is made of a soft, optionally plumbiferous plastic.

One method of carrying out the invention is described in detail below with reference to drawings, which illustrate only one specific embodiment, in which

Fig. 1 illustrates the air purifier according to the invention, and

Fig. 2 illustrates a voltage multiplier forming part of the air purifier.

The air purifier according to the invention operates in accordance with the regeneration principle. It comprises a radiating means 1 such as a mercury lamp, emitting both electrons and X-rays. By applying a voltage of 220 VAC, the X-ray wave length is in the range of 300 – 400 nm. By applying a voltage of 8000 V, the wave length range is increased to about 165 nm. At a wave length of 210 nm a destruction of DNA molecules and consequently a destruction of germs and viruses takes place. At a wave length of 250 nm a cell division takes place so quickly that the cells produced cannot survive. By the electron bombardment the oxygen, hydrogen and nitrogen bound to heavy molecules such as sulphur, chlorine or metals, are liberated. The heavy molecules are detected and captured by the chamber material, while the liberated oxygen molecules produce ozone molecules. These ozone molecules are, however, positively charged due to the fact that the heavy particles detected and captured by the chamber material are negatively charged (the chamber material must be non-conductive). An electron-cloud forming member, preferably a capacitor 4, is placed behind the radiating means 1, said capacitor generating negatively charged $O_3$ molecules. When a positively charged $O_3$ molecule meets a negatively charged $O_3$ molecule, the oppositely charged molecules recombine to produce $O_2$ molecules.

The tension across the capacitor 4 is for instance of the magnitude 8000 V, which corresponds substantially to the voltage necessary for the radiating means 1 to emit X-rays having wave lengths down to 165 nm.

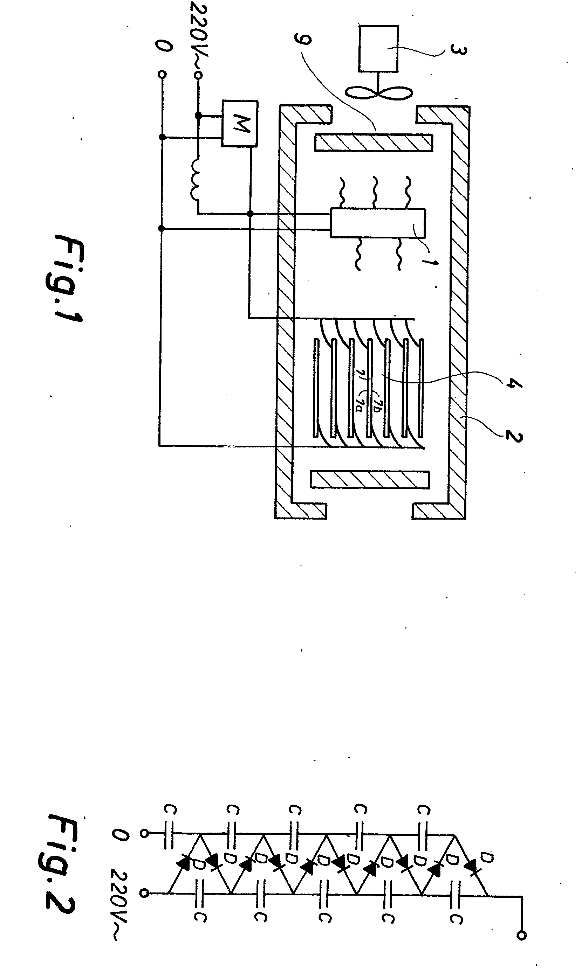

Fig. 2 shows a voltage multiplier M comprising a sufficient number of capacitors C mutually connected through rectifier diodes in such a manner that the charge can only be transmitted in one direction. Fig. 2 only shows 10 capacitors.

The capacitor 4 is composed of plates 7 having metal coatings 7a and 7b on both sides. The surfaces of these coatings are suited for emission of electrons. The number of plates 7 may be varied. The surface area of each plate is $10 \times 10$ cm². The mutual distance between the plates is for instance 1 cm. The casing 2 surrounding the radiating means 1 and the capacitor 4 is composed of a soft, optionally plumbiferous plastic. A separate plumbiferous coating may also be provided on the outside of the casing 2. A blower 3 is situated at an inlet opening 9 constructed in such a manner that radiation direct to the surrounding is impossible.

An air purifier with a power consumption of 80 W can treat 60 m³ per hour.

A choke coil is inserted between the output of the voltage multiplier M and the phase terminal. The power supplied at the high voltage is relatively low. Furthermore, a DC-discharge from the multiplier only occurs at every other half wave of the voltage of the mains.

The air purifier according to the invention is inexpensive to produce. The most expensive component is the casing.

By using the air purifier according to the invention the oxygen content in a room is increased no more than 6 – 8%.

The air purifier, which may be varied in many ways without deviating from the scope of the invention, is mainly intended for aircrafts, automobiles or hospitals. The purifier may, however, also be used industrially or in offices. When used industrially, the air purifier improves the air for the employees. It is easy to mount, since it does not require ventilating ducts to the surroundings. Furthermore, no thermal loss occurs. In slaughterhouses it increases the keeping quality of the meat since only viruses and bacteria are killed.

## Claims

1. An air purifier of the regenerating type which comprises a radiating means (1), surrounded by a casing (2) and capable of emitting electrons and X-rays of a wavelength of at least 165 nm and producing ozone molecules in the air stream, and in which the air flows during use of the purifier through an electric field in an electroncloud forming member (4) arranged within the casing (2) downstream of the radiating means for generating negatively charged ozone molecules in the stream as the latter flows through the electric field, characterized by the casing being a negative charge absorbing, non-conductive casing.

2. An air purifier as claimed in claim 1, characterised by the non-conductive casing (2) being of a soft, optionally plumbiferous plastic.

3. An air purifier as claimed in claim 1 or 2, characterised in that both the radiating means (1) and the electron-cloud forming member (4) are actuable by a voltage multiplier connectible to the mains.

4. An air purifier as claimed in claims 1–3, characterised by the electron-cloud forming member (4) being a capacitor.

## Patentansprüche

1. Luftreiniger des Regeneriertyps mit einer von einem Gehäuse umgegebenen ausstrahlenden Einrichtung, die imstande ist, Elektronen und Röntgenstrahlen mit einer Wellenlänge von mindesetns 165 nm auszustrahlen und Ozonmoleküle im Luftstrom zu erzeugen, und in der die Luft während des Anwendens des Reinigers durch ein elektrisches Feld in einer Elektronenhülle bildenden Vorrichtung strömt, wobei die Vorrichtung im Gehäuse stromabwärts im Verhältnis zur ausstrahlenden Einrichtung angeordnet ist, die zum Erzeugen negativ geladener Ozonmoleküle im Strom während des Durchströmens durch das elektrische Feld vorgesehen ist, dadurch gekennzeichnet, daß das Gehäuse ein negativ ladungsabsorbierendes, nichtleitendes Gehäuse ist.

2. Luftreiniger nach Anspruch 1, dadurch gekennzeichnet, daß das nichtleitende Gehäuse (2) aus einem weichen, ggf. bleihaltigen Kunststoff ist.

3. Luftreiniger nach Anspruch 1 oder 2, dadurch gekennzeichnet, daß sowohl die ausstrahlende Einrichtung (1) als auch die Elektronenhülle bildende Vorrichtung (4) durch einen mit dem Starkstromnetz ankuppelbaren Verstärker aktivierbar sind.

4. Luftreiniger nach den Ansprüchen 1 bis 3, dadurch gekennzeichnet, daß die Elektronenhülle bildende Vorrichtung (4) ein Kondensator ist.

## Revendications

1. Purificateur d'air du type régénérateur, comprenant des moyens de radiation (1), entourés d'un coffret (2) et agencés pour émettre des électrons et des rayons X d'une longueur d'onde d'au moins 165 nm et produire des molécules d'ozone dans le courant d'air, et dans lequel, lorsque le purificateur est en marche, l'air circule à travers un champ électrique dans un élément (4), formant un nuage d'électrons, disposé dans le coffret (2) en aval des moyens de radiation, pour engendrer des molécules d'ozone chargées négativement dans le courant d'air lorsque celui-ci traverse le champ électrique, caractérisé par le fait que le coffret est un coffret non conducteur absorbant les charges négatives.

2. Purificateur d'air selon la revendication 1, caractérisé par le fait que le boîtier non conducteur (2) est en matière plastique tendre, par exemple plombifère.

3. Purificateur d'air selon l'une des revendications 1 et 2, caractérisé par le fait que les moyens de radiation (1) et l'élément un nuage d'électrons (4) sont tous deux commandables par un multiplicateur de tension raccordable au réseau.

4. Purificateur d'air selon l'une des revendications 1 à 3, caractérisé par le fait que l'élément formant un nuage d'électrons (4) est un condensateur.

*Fig.1*

*Fig.2*